# EUROPÄISCHE PATENTSCHRIFT

(11) **EP 1 135 460 B1**
(45) Veröffentlichungstag und Bekanntmachung des Hinweises auf die Patenterteilung: **14.05.2003**
(21) Anmeldenummer: 99961053.8
(22) Anmeldetag: 02.12.1999
(51) Int. Cl.: C12M 1/34, C12M 1/12

(54) **VERFAHREN UND VORRICHTUNG ZUR SELEKTION BESCHLEUNIGTER PROLIFERATION LEBENDER ZELLEN IN SUSPENSION**
METHOD AND DEVICE FOR SELECTING ACCELERATED PROLIFERATION OF LIVING CELLS IN SUSPENSION
PROCEDE ET DISPOSITIF POUR LA SELECTION DE LA PROLIFERATION ACCELEREE DE CELLULES VIVANTES EN SUSPENSION

(30) Priorität: 04.12.1998 DE 19856136
(43) Veröffentlichungstag der Anmeldung: 26.09.2001
(73) Patentinhaber: INSTITUT PASTEUR, 75724 Paris Cédex 15 (FR); Mutzel, Rupert, 12247 Berlin (DE)
(72) Erfinder: MUTZEL, Rupert, D-78464 Konstanz (DE); MARLIERE, Philippe, F-91450 Etiolles (FR)
(74) Vertreter: VOSSIUS & PARTNER
(86) Internationale Anmeldenummer: EP9909422
(87) Internationale Veröffentlichungsnummer: WO00034433

(56) Entgegenhaltungen:
- EP-A- 0 620 273
- FR-A- 1 064 614
- US-A- 2 147 271
- WYSS C: "SELECTED LOW-COHESION VARIANTS OF ACTINOBACILLUS-ACTINOMYCETEMCOMITANS AND HAEMOPHILUS-APHROPHILUS LACK DISTINCT ANTIGENS RECOGNIZED BY HUMAN ANTIBODIES" ARCHIVES OF MICROBIOLOGY 1989, Bd. 151, Nr. 2, 1989, Seiten 133-136, XP002136552 ISSN: 0302-8933

## Beschreibung

Die vorliegende Erfindung betrifft ein Verfahren sowie eine Vorrichtung zur Selektion beschleunigter Proliferation lebender Zellen in Suspension.

Herkömmlicherweise wird zwischen seriellen und kontinuierlichen Kulturmethoden unterschieden. Bei der Technik der seriellen Kultur werden Kulturgefäße mit sterilem Wachstumsmedium mit einer Fraktion einer Kultur beimpft, die unter den gleichen Wachstumsbedingungen angezogen wurde. Dieser Zyklus wird wiederholt, wenn die neue Kultur gewachsen ist (Lenski, R.E. und Travisano, M. (1994): Dynamics of adaption and diversification: A 10,000-generation experiment with bacterial populations. Proc. Natl. Acad. Sci. USA 91, 6808-6814). Die in der Literatur beschriebenen Experimente zur Proliferation mikrobieller Organismen über die längsten Zeiträume wurden mit dieser Methode durchgeführt (Lenski und Travisano, a.a.O.). Bei kontinuierlichen Kulturmethoden (Dijkuizen, D.E. (1993): Chemostats used for studying natural selection and adaptive evolution. Meth. Enzymol. 224, 613-631), wird eine Kultur kontinuierlich nach einem vorgewählten Regime mit frischem Wachstumsmedium verdünnt. In der Literatur sind solche Experimente nur von begrenzter Dauer beschrieben (Dijkuizen, a.a.O.).

Die vorstehend beschriebenen herkömmlichen Techniken haben insbesondere den Nachteil, daß bisher kein Verfahren zur kontinuierlichen Kultur beschrieben wurde, das die permanente Proliferation von Organismen in Suspension gewährleistet. Alle beschriebenen Apparaturen selektionieren verdünnungsresistente (statische) Varianten, welche innere Oberflächen der Apparatur besiedeln (Chao, L. and Ramsdell, G. (1985): The effects of wall populations on coexistence of bacteria in the liquid phase of chemostat cultures. J. Gen. Microbiol. 131, 1229-1236). Diese Varianten bilden eine Subpopulation, die den adaptiven Zwängen entkommt, die auf die Organismen in Suspension wirken. Kontinuierliche Kulturen mit konstanter Zelldichte, wie z.B. Turbidostat sind besonders anfällig für eine Invasion durch verdünnungsresistente Varianten und können nur über relativ kurze Zeiträume (etwa 200 Generationen) durchgeführt werden. Die Literatur beschreibt diese Schwierigkeiten, hat aber bisher nur untaugliche Lösungsmöglichkeiten angeboten. Aus diesem Grunde werden derartige Verfahren für wissenschaftliche und industrielle Ziele praktisch nicht angewendet, obwohl ihr Potential sehr früh erkannt wurde (Monod, J. (1950): La technique de la culture continue. Théorie et applications. Arin. Inst. Pasteur 79, 390-410; Novick, A. and Szilard, L. (1950): Description of the chemostat. Science 112, 715-716). Die serielle Kultur, die man mit der periodischen Erneuerung der Kulturapparatur - in diesem Fall eines einfachen Kulturgefäßes - beschreiben kann, vermeidet die Invasion mit solchen verdünnungsresistenten Varianten. Sie ist jedoch arbeitsaufwendig, d.h. personalintensiv und durch wiederholte Manipulation unter Bedingungen, die absolute Sterilität erfordern, kontaminationsanfällig (Lenski und Travisano, a.a.O.). Robotisierung der seriellen Kultur in einer sterilen Umgebung könnte diese Risiken reduzierten. Sie würde jedoch mit einem großen Aufwand an Kulturgefäßen erkauft und wäre durch die Aufrechterhaltung der mechanischen Präzision des Roboters und die Aufrechterhaltung der sterilen Umgebung limitiert.

Die US-A-2,147,271 betrifft eine Vorrichtung zur Produktion von reiner Kulturhefe in großem Umfang unter sterilen Bedingungen. Die Vorrichtung weist einen Sterilisator und einen Reaktor auf, die jeweils mit einem Leitungssystem in Verbindung stehen. Das Leitungssystem weist eine Luftzuführung auf, die sowohl mit dem Sterilisator wie auch mit dem Reaktor in Verbindung steht. Darüber hinaus sind Entlüftungsleitungen und Verbindungsleitungen vorgesehen, so dass der Inhalt aus dem Sterilisator in den Reaktor überführt werden kann und der Inhalt aus dem Reaktor zur weiteren Verwendung abgegeben werden kann.

Es ist daher eine Aufgabe der vorliegenden Erfindung, ein verbessertes Verfahren und eine verbesserte Vorrichtung zur Selektion beschleunigter Proliferation lebender Zellen in Suspension zur Verfügung zu stellen. Diese Aufgabe wird mit den Merkmalen der Patentansprüche gelöst.

Dabei geht die Erfindung von den Grundgedanken aus, daß die Vorrichtung eine Suspension von Zellen dauernd in Proliferation hält. In keinem Teil der Apparatur darf eine verdünnungsresistente Variante akkumulieren. Ihre Funktion wird durch die Steuerung von Flüssigkeitsströmen (Fluidics) gewährleistet. Unter der Voraussetzung, daß die regelmäßige Nachlieferung von Flüssigkeiten, wie z.B. Mährmedien und Waschlösungen, und eine kontinuierliche Versorgung mit sterilen Gasen, wie z.B. Luft gewährleistet wird, muß die Apparatur über unbegrenzte Zeitdauer autonom arbeiten. Verschiedene Kulturregime, wie z.B. Chemostat oder Turbidostat, können angewendet werden. Bei Bedarf können bestimmte Bestandteile von Zellen durch die Wirkung geeigneter Lösungen abgetrennt oder isoliert werden. Bei Bedarf können mehrere der Apparaturen so miteinander verbunden werden, daß der Inhalt oder ein Teil des Inhalts einer Apparatur in eine andere überführt werden kann.

Konkret wird die Anforderung, daß eine Population von Zellen unter kontinuierlichen Kulturbedingungen ausschließlich in Suspension profiliert, durch vorzugsweise periodische Überführung der Organismensuspension aus einem ersten Kulturgefäß in ein zweites Kulturgefäß erfüllt. Nach der Überführung wird das erste Kulturgefäß einer sterilisierenden Behandlung unterzogen, das sterilisierende Agens gegebenenfalls neutralisiert, und das erste Kulturgefäß ist dann wiederum bereit für die Rücküberführung der Kultur aus dem zweiten Kulturgefäß, das anschließend der Sterilisation und Neutralisation unterzogen wird. Dieser Ablauf stellt dabei sicher, daß (i) die Population von Organismen in Suspension zu jedem Zeitpunkt erhalten bleibt und (ii) alle verdünnungsresistenten Varianten in irgend einem Teil der Apparatur während jedem der Zyklen zerstört werden.

Das Verfahren der alternierenden Sterilisierung der Kulturgefäße selektioniert vorzugsweise direkt und regelmäßig gegen variante Organismen, die Oberflächen in der Apparatur besiedeln und verhindert die Proliferation und Adaption einer statischen, verdünnungsresistenten Population. Jede Vorrichtung zur kontinuierlichen Kultur von Organismen kann als Apparatur betrachtet werden, in der die natürliche Selektion Mutanten bevorzugt, die der Verdünnung widerstehen. Das beschriebene Verfahren bietet der kultivierten Population als einzige Möglichkeit durch eine Erhöhung der Proliferationsrate in Suspension der Verdünnung zu widerstehen. Im Unterschied zu einem Fermentationsverfahren beschreibt die Erfindung ein Verfahren der automatisierten Genetik, das gleichzeitig statische Varianten gegenselektioniert und dynamische Varianten bevorzugt, die immer besser an die Kulturbedingungen angepaßt sind.

Somit hat die vorliegende Erfindung gegenüber dem Stand der Technik insbesondere den Vorteil, daß ein Regime konstanter Zelldichte (Turbidostat) über unbegrenzte Zeiträume aufrechterhalten werden kann und daß die Wachstumsrate natürlich vorkommender oder gentechnisch veränderter Zellen erhöht werden kann. Ein Beispiel für industrielle Anwendungen ist die Anreicherung natürlicher Varianten, die ein chemisches Produkt (wie z.B. ein Zwischenprodukt chemischer Synthese oder ein Umweltgift) metabolisieren können. Ein weiterer Fall wäre die Verbesserung eines Enzyms oder eines Stoffwechselweges: wenn die Umwandlung eines Substrats in ein Produkt der limitierende Schritt im Stoffwechsel einer Zelle ist und die Zelle mit diesem Substrat im Überschuß versorgt werden kann, so wird die kontinuierliche Kultur unter den beschriebenen Bedingungen zu einer Zunahme der Wachstumsrate führen, die aus einer erhöhten Umsatzrate des Substrats resultiert, und diese erhöhte Umsatzrate resultiert aus der Fixierung von aufeinanderfolgenden Mutationen in dem Gen oder den Genen für die Enzyme, die für den zu leistenden Umsatz des Substrats der Selektion unterliegen.

Ein weiterer Vorteil der vorliegenden Erfindung ist, daß mehrere verschiedene Wachstumsmedien der Organismensuspension durch die Apparatur zugeführt werden können. Dies erlaubt, vielfache oder alternierende Adaption durchzuführen und die metabolische Kapazität der kultivierten Organismen zu diversifizieren. Eine vorgewählte Zelldichte kann von anderen Variablen als der Zufuhr frischen Mediums abhängig gemacht werden. Das Erreichen dieser Dichte kann die Wirkung chemischer und physikalischer Agenzien konditionieren, deren Toxizität derart eingestellt ist, daß die Population der Organismen ständig an ihrer Toleranzgrenze ist oder daß zunehmend resistente Varianten selektioniert werden. Zudem können durch die Zufuhr von Detergenzien oder Lösungsmitteln bestimmte Bestandteile von Zellen extrahiert werden. Insbesondere können genetisches Material wie Plasmide oder Viren automatisch extrahiert werden, wobei die Wirtszellen zerstört werden. Auf der anderen Seite können bestimmte Agenzien zugeführt werden, die die Zellen kompetent für genetische Transformation oder Infektion mit natürlichen oder synthetisch hergestellten Nucleinsäuren machen. Dieses genetische Material kann dann in die Population eingeführt werden. Ganz allgemein können zwei oder mehrere Apparaturen miteinander verbunden werden. Damit können verschiedene Organismen automatisch in Kontakt gebracht werden und es können automatisch verschiedenste genetische Materialien, wie z.B. konjugative Episomen, Phagen, Transposons, usw. eingeführt werden.

Die vorliegende Erfindung wird im folgenden anhand einer bevorzugten Ausführungsform beispielhaft unter Bezugnahme auf die Zeichnungen beschrieben. Darin zeigen:
- Fig. 1: eine erfindungsgemäße Vorrichtung zur Selektion beschleunigter Proliferation lebender Zellen in Suspension in einer Ausgangsposition, in der sich die Zellsuspensionen in einem ersten Kulturgefäß befindet;
- Fig. 2: die Vorrichtung von Fig. 1, bei der sich die Zellsuspension in einem zweiten Kulturgefäß befindet;
- Fig. 3: die Vorrichtung von Fig. 2, wobei das erste Kulturgefäß ein sterilisierendes Agens aufweist;
- Fig. 4: die Sterilisierung einzelner Leitungsabschnitte;
- Fig. 5: die Vorrichtung beim Entfernen des sterilisierenden Agens aus dem ersten Kulturgefäß sowie den dazugehörigen Leitungsabschnitten;
- Fig. 6-8: Schritte zur Entfernung und Neutralisierung von Resten des sterilisierenden Agens aus dem ersten Kulturgefäß und den dazugehörigen Leitungsabschnitten mit einer Waschlösung;
- Fig. 9: die Vorrichtung von Fig. 3, bei der das erste Kulturgefäß und die dazugehörigen Leitungsabschnitte sterilisiert und neutralisiert sind; und
- Fig. 10-16: die entsprechend umgekehrte Vorgehensweise zur Überführung der Kultur aus dem zweiten Kulturgefäß in das erste Kulturgefäß.

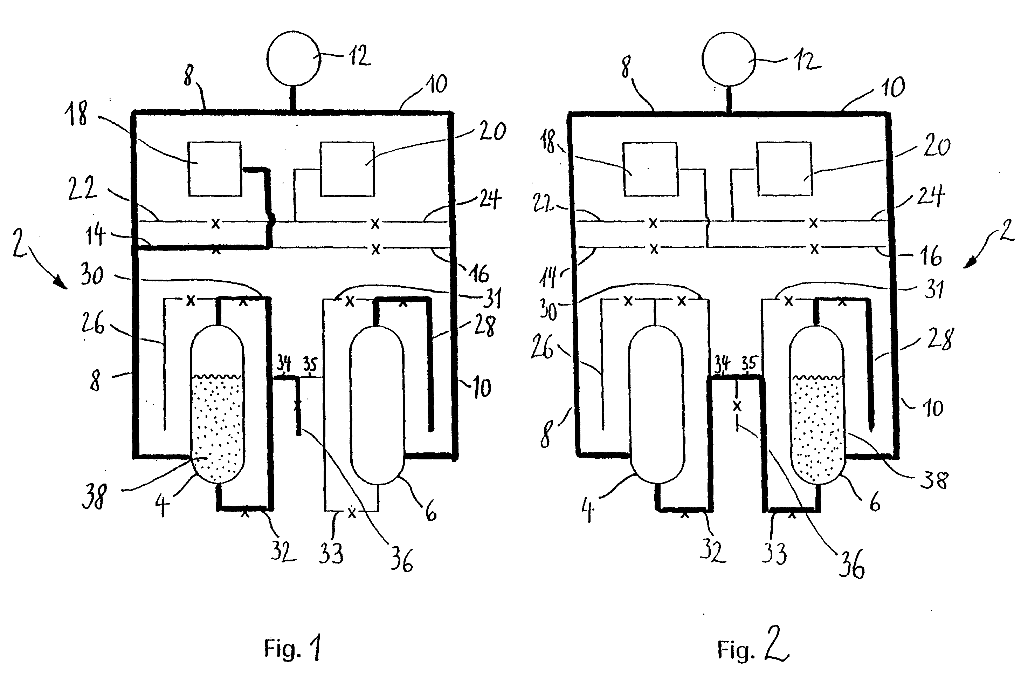

Die in den Figuren 1 bis 16 dargestellte Vorrichtung zur Selektion beschleunigter Proliferation lebender Zellen in Suspension kann auch als Kulturapparatur 2 bezeichnet werden. Die Kulturapparatur 2 ermöglicht die Proliferation von Zellen in Suspension über unbegrenzte Zeiträume. Durch die natürliche Selektion werden genetische Varianten angereichert, die zunehmend besser an die gewählten Kulturbedingungen angepaßt sind. Die verwendeten Organismen können prokaryontisch oder eukaryontisch sein. Ferner können die verwendeten Organismen natürlich vorkommend oder genetisch verändert sein. Konstante (Kubitschek, H.E. (1970): Introduction to research with continuous cultures. Prentice-Hall; Pirt, S.J. (1975): Principles of microbe and cell cultivation. Blackwell), periodische (Pirt, a.a.O.) oder konditionale (Bryson, V. (1952): The turbidostatic selector - a device for automated isolation of bacterial variants. Science 116, 48-51; Fraleigh, S.P., Bungay, H.R. and Clesceri, L.S. (1989): Continuous culture, feedback and auxostats. TIBTech. 7, 159-164) Kulturbedingungen können verwendet werden. Physikalische und chemische Charakteristika der benutzten Kulturmedien können vom Benutzer gewählt werden.

Der Aufbau und das Funktionsschema der Kulturapparatur 2 wird nachstehend anhand der Figuren 1 bis 16 beschrieben. Die Kulturapparatur 2 weist im wesentlichen ein erstes Kulturgefäß 4 und ein zweites Kulturgefäß 6 auf. Die beiden Kulturgefäße 4 und 6 sind über Leitungen 8 bzw. 10, die vorzugsweise in den unteren Bereich der Kulturgefäße 4 bzw. 6 münden, mit einer unter Überdruck stehenden Gasversorgung 12 verbunden. Des weiteren sind die Kulturgefäße 4 und 6 über Leitungen 14 und 16 jeweils mit einer ebenfalls unter Überdruck stehenden Mediumsquelle 18 verbunden. Die Leitungen 14 und 16 münden vorzugsweise von der Mediumsquelle 18 kommend jeweils in die Leitungen 8 bzw. 10 zur Verbindung der Gasversorgung 12 mit den beiden Kulturgefäßen 4 bzw. 6. Darüber hinaus ist eine unter Überdruck stehende Quelle 20 für ein sterilisierendes Agens 21 (z.B. NaOH) vorgesehen, die mittels Leitungen 22 und 24 mit den jeweiligen Kulturgefäßen 4 bzw. 6 in Verbindung steht. Vorzugsweise münden die Leitungen 22 und 24 in die Leitungen 8 und 10, wie vorstehend für die Leitungen 14 und 16 beschrieben.

An den Kulturgefäßen 4 und 6 ist ferner jeweils eine Ablaßleitung 26 bzw. 28 vorgesehen, um überschüssiges sterilisierendes Agens 21 bzw. Waschlösungen 19 während der Sterilisation und Neutralisation des jeweiligen Kulturgefäßes 4 oder 6 abzuführen. Des weiteren sind Verbindungsleitungen 30, 31, 32 und 33 zwischen den beiden Kulturgefäßen 4 und 6 vorgesehen, um die beiden Kulturgefäße 4 und 6 miteinander in Verbindung zu bringen. Vorzugsweise weisen mindestens zwei der Verbindungsleitungen einen gemeinsamen Abschnitt 34 und 35 auf, an dem eine Ablaßleitung 36 vorgesehen ist, die der vollständigen Entleerung eines der beiden Kulturgefäße 4 oder 6 oder der Entnahme der Kultur 38 oder eines Teils davon dient.

Zur Steuerung des erfindungsgemäßen Verfahrens zur Selektion beschleunigter Proliferation lebender Zellen in Suspension sind ferner in den Leitungen oder Leitungsabschnitten 14, 16, 22, 24, 30, 31, 32, 33, 34, 35 und 36 Ventile (schematisch dargestellt) vorgesehen. Die Ventile können beispielsweise mechanisch betätigt werden oder aber vorzugsweise unter Verwendung einer nicht dargestellten Steuereinrichtung elektrisch bzw. elektronisch vorzugsweise automatisch angesteuert werden.

Im folgenden wird anhand der Figuren 1 bis 16 das Funktionsschema des erfindungsgemäßen Verfahrens mit der vorstehend beschriebenen Kulturapparatur 2 näher erläutert. Dazu ist anzumerken, daß abgesperrte Leitungsabschnitte dünn und durchströmte bzw. durchströmbare Leitungsabschnitte oder Leitungen dick eingezeichnet sind. Die Ventile sind lediglich schematisch dargestellt, so daß aufgrund ihrer Darstellungsform kein Rückschluß auf die verwendete Ventilart gemacht werden sollte. Ebenso ist der Leitungsplan sowie die Ventilanordnung lediglich schematisch. Geeignete Ventile sowie die bestmögliche Leitungsführung sowie Ventilanordnung können je nach Anwendungsfall variieren.

Die Figuren 1 bis 16 stellen die wichtigsten aufeinanderfolgenden Etappen im Kultivierungs/Sterilisierungszyklus dar. Gemäß Fig. 1 befindet sich die Zellsuspension unter vorgewähltem Kulturregime (Chemostat, Turbidostat) im ersten Kulturgefäß 4. Die Ventile sind dabei so angesteuert, daß das erste Kulturgefäß 4 sowohl mit der Gasversorgung 12 wie auch mit der Mediumsquelle 18 in Verbindung steht, um der Kultur 38 regelmäßig Flüssigkeiten, wie z.B. Nährmedien und Waschlösungen nachzuliefern und eine kontinuierliche Versorgung mit sterilen Gasen, wie z.B. Luft, zu gewährleisten. Die Verbindung des ersten Kulturgefäßes 4 zur Quelle 20 für das sterilisierende Agens 21 ist durch das entsprechende Ventil unterbrochen. Das zweite Kulturgefäß 6 hat eine Verbindung zur Gasquelle 12. Durch die Leitung 36 kann die Kultur 38 oder Teile davon entnommen werden.

Entsprechend Fig. 2 ist die Kultur 38 über die Leitungen 32, 34, 35 und 33 in das zweite Kulturgefäß 6 überführt worden, wobei das Öffnen der Leitung 28 einen Druckausgleich gewährleistet. Alle von der Mediumsquelle 18 und der Quelle 20 des sterilisierenden Agens zu den Kulturgefäßen 4 und 6 führenden Leitungen sind abgesperrt.

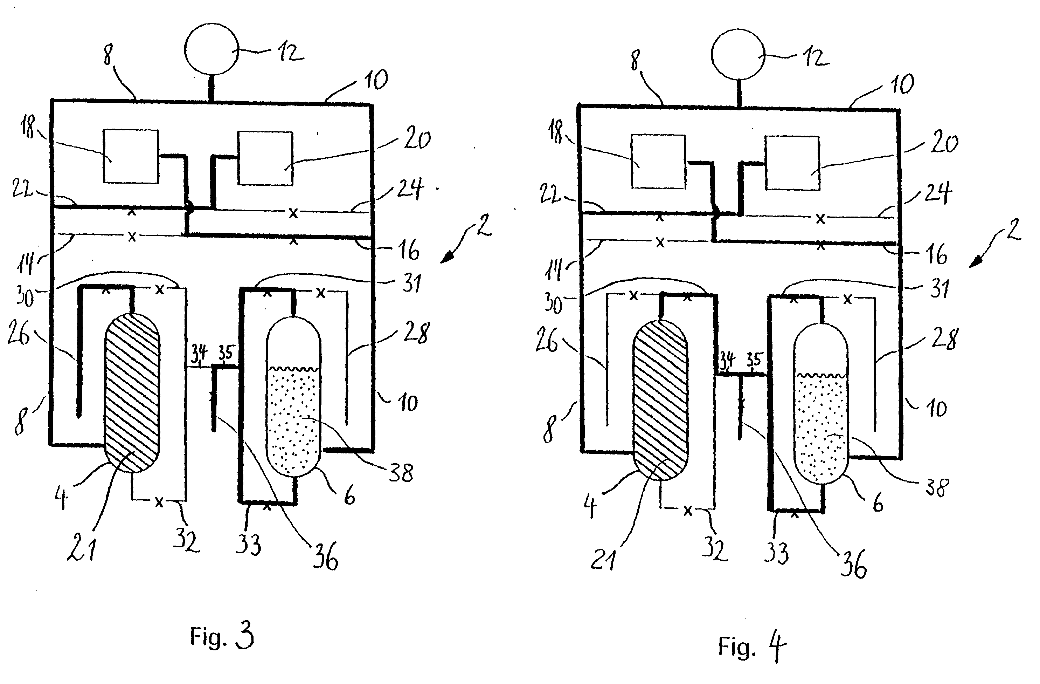

In der in Fig. 3 dargestellten Situation ist nach Absperren der Leitungen 32, 34, 35 und 33 und Öffnen der Leitungen 22 und 26 das erste Kulturgefäß 4 mit sterilisierendem Agens 21 gefüllt worden; ein Überschuß an sterilisierendem Agens 21 wird über die Ablaßleitung 26 abgeführt. Die Kultur 38 im zweiten Kulturgefäß 6 kann nach Herstellung der Verbindung 16 zwischen der Mediumsquelle 18 und dem Kulturgefäß 6 sowie Absperren der Leitung 28 und Öffnen der Leitungsabschnitte 31, 33, 35 und 36 wieder regelmäßig mit Medium versorgt werden.

Fig. 4 zeigt die Sterilisierung der Leitungen 30, 34 und 36 durch sterilisierendes Agens 21 nach Öffnen der entsprechenden Leitungsabschnitte und Schließen der Ablaßleitung 26.

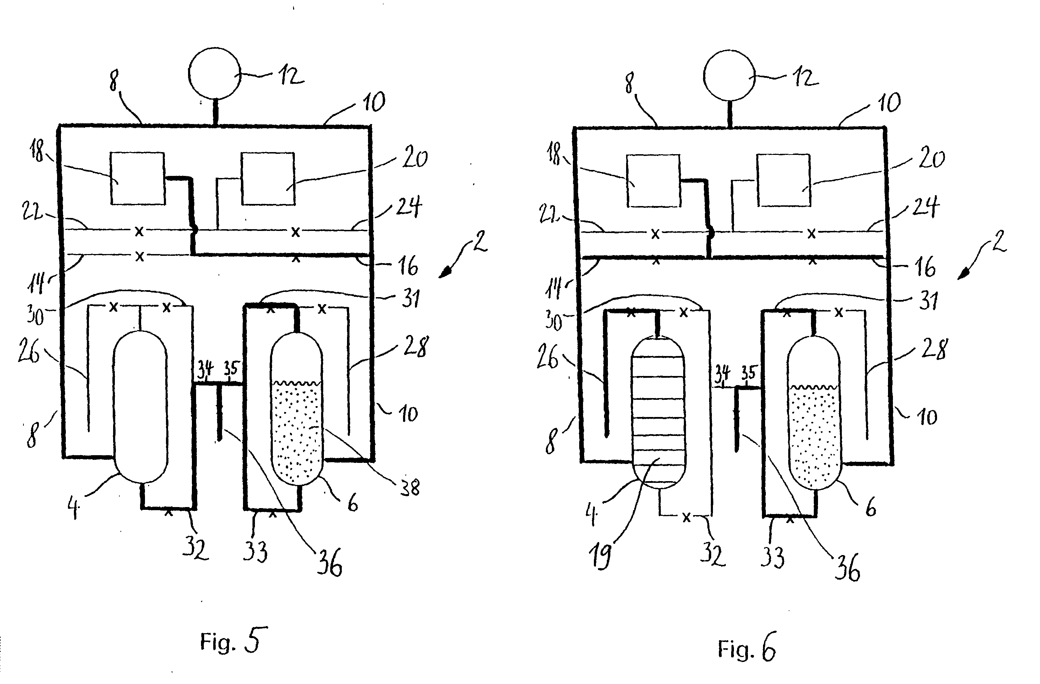

Das sterilisierende Agens 21 wird nun gemäß Fig. 5 nach Absperren der Leitungen 22, 26 und 30 sowie Öffnen der Leitung 32 aus dem ersten Kulturgefäß 4 entfernt.

Die Figuren 6 bis 8 zeigen vorzugsweise optionale Schritte zur Entfernung und Neutralisierung von eventuell verbliebenen Resten des sterilisierenden Agens 21 aus dem ersten Kulturgefäß 4 und den dazugehörigen Leitungsabschnitten durch frisches Medium:

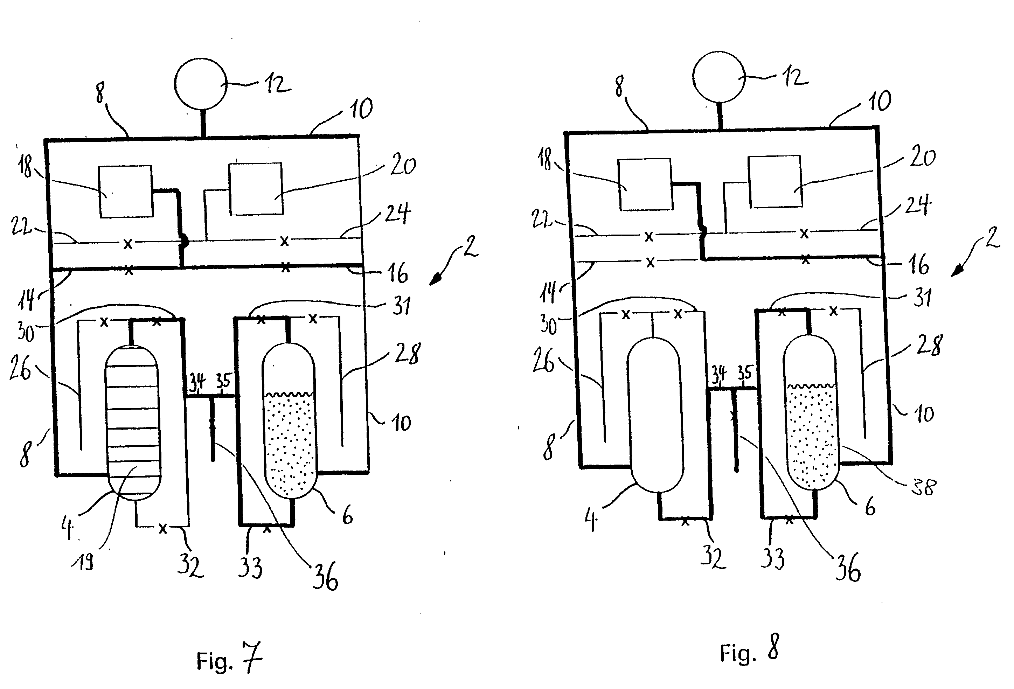

In Fig. 6 wird das erste Kulturgefäß 4 nach Schließen der Leitungen 32 und 36 und Öffnen der Leitung 26 durch Öffnen der Leitung 14 mit Medium gefüllt, überschüssiges Medium wird durch die Ablaßleitung 26 abgeführt. Analog der Situation in Fig. 4 werden nun die Leitungen 30, 34 und 36 mit Medium durchspült (Fig. 7), um dann das Medium analog der Situation in Fig. 5 aus dem Kulturgefäß zu entfernen (Fig. 8).

Nach dem Öffnen der Leitung 26 und Schließen der Leitungen 32 und 34 des Kulturgefäßes 4 ergibt sich eine Situation, die der in Fig. 1 dargestellten spiegelsymmetrisch ist, wobei sich die Zellsuspension nun im zweiten Kulturgefäß 6 befindet. Durch die Leitung 36 kann die Kultur 38 oder Teile davon entnommen werden.

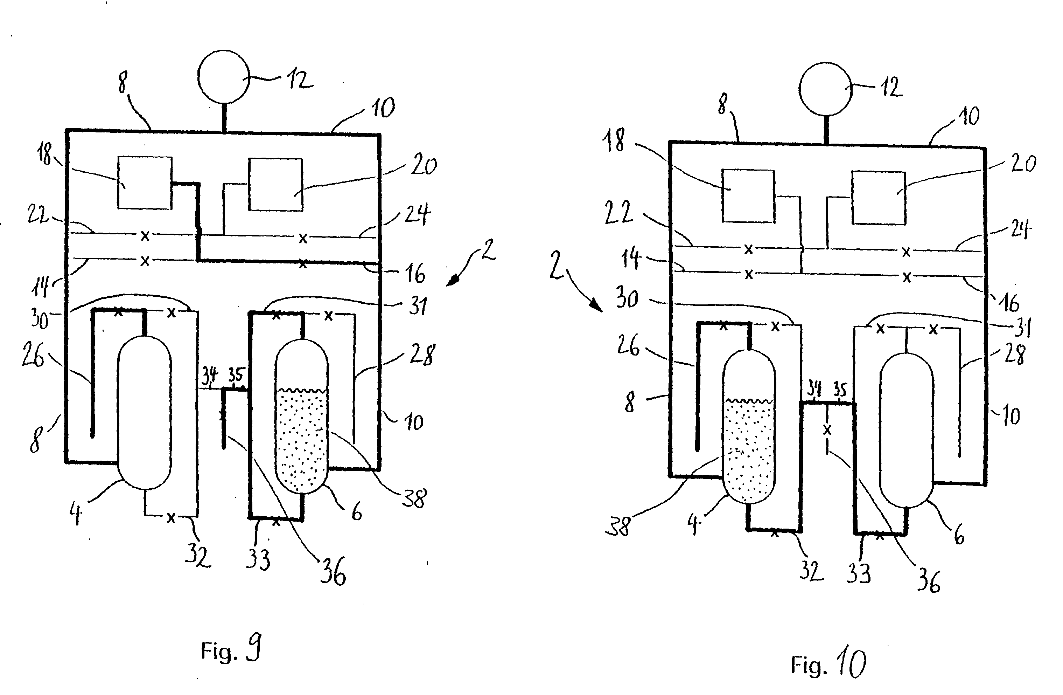

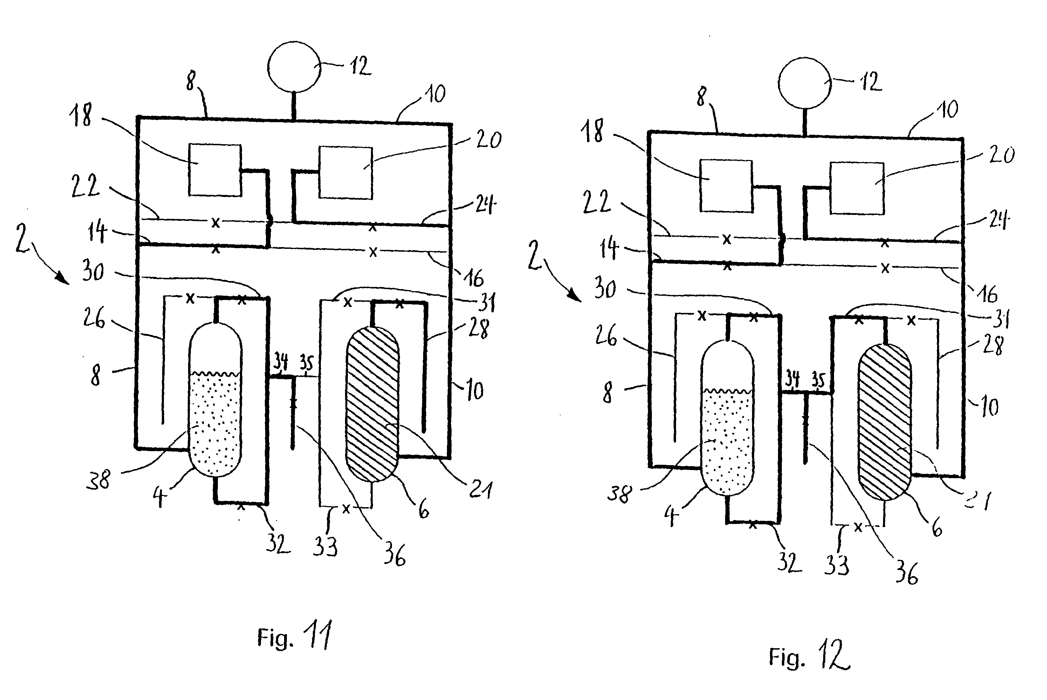

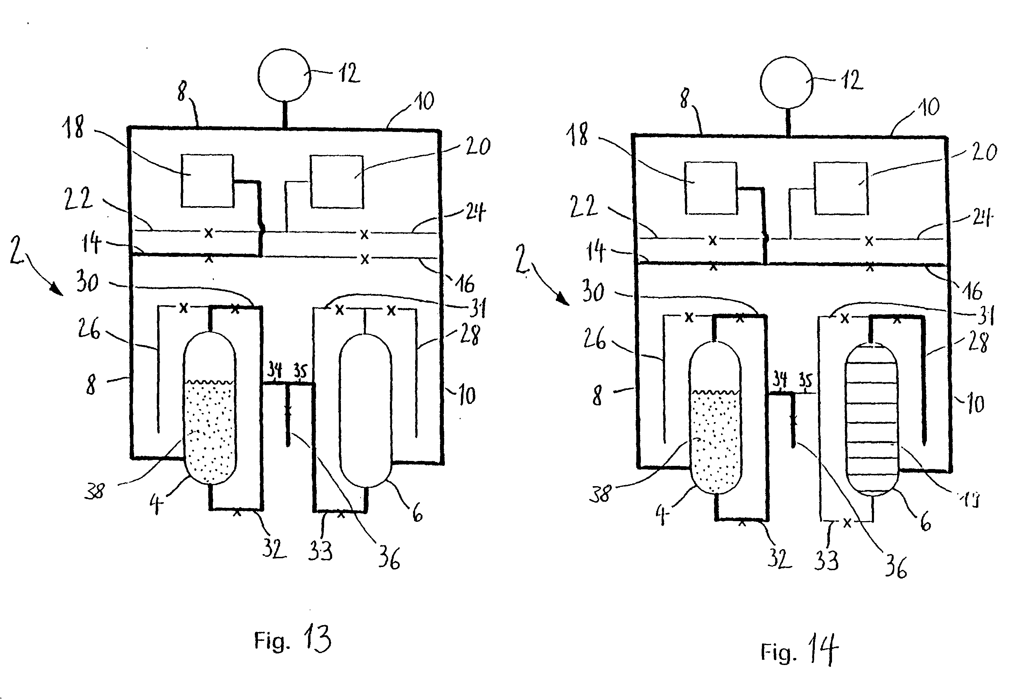

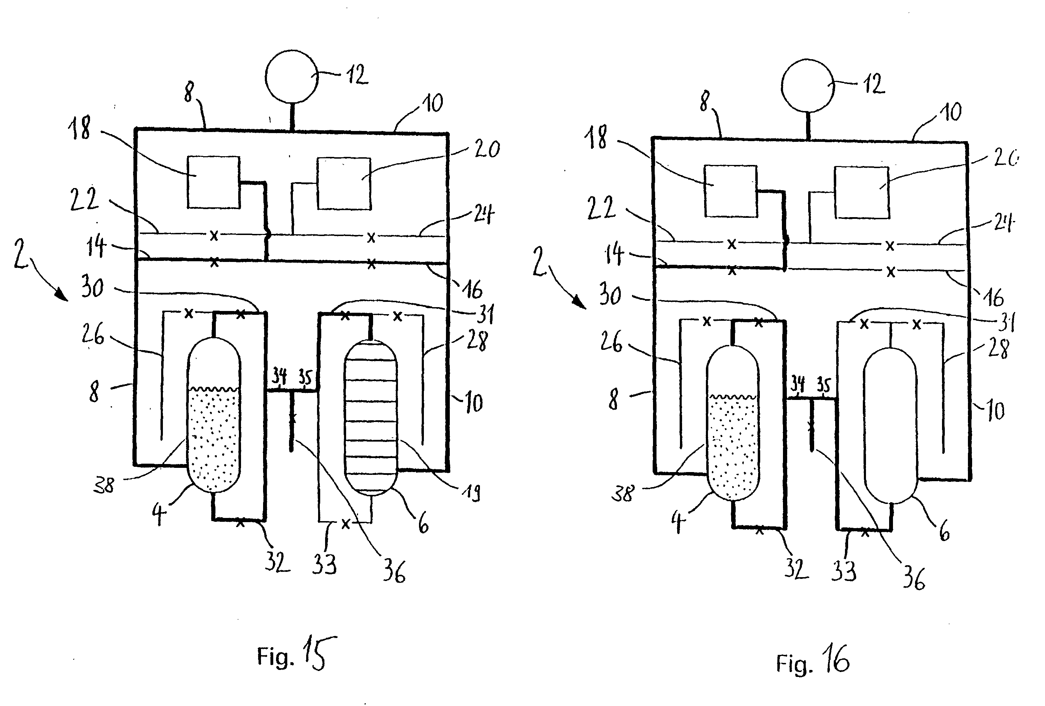

Die Figuren 10 bis 16 stellen die entsprechenden symmetrischen Schritte dar, um zur Ausgangssituation in Fig. 1 zurückzukehren, nämlich: Fig. 10, Überführung der Kultur 38 aus dem zweiten Kulturgefäß 6 in das erste Kulturgefäß 4; Fig. 11, Sterilisieren des zweiten Kulturgefäßes 6 mit sterilisierendem Agens 21; Fig. 12, Sterilisieren der Leitungsabschnitte 31, 35 und 36; Fig. 13, Entfernen des sterilisierenden Agens 21 über die Leitungsabschnitte 33, 35 und 36; Fig. 14, Waschen des Kulturgefäßes 6 und der Ablaßleitung 28 mit frischem Medium; Fig. 15, Waschen der Leitungen 31, 35 und 36 durch frisches Medium und Fig. 16, Entfernen des zum Waschen verwendeten Mediums über die Leitungen 33, 35 und 36. Öffnen der Leitung 14 zum Kulturgefäß 4, Öffnen der Leitungen 30 und 34 und Schließen der Leitungen 26 sowie Öffnen der Leitung 28 und Schließen der Leitungsabschnitte 33 und 35 stellt die Ausgangssituation von Fig. 1 wieder her.

Anzumerken ist, daß zu jedem Zeitpunkt des vorstehend beschriebenen Verfahrens, zu dem die Ablaßleitung 36 nicht von sterilisierendem Agens durchströmt wird, ein Abführen der Kultur 38 durch den jeweiligen Verbindungsabschnitt und die Ablaßleitung 36 gewährleistet ist.

Des weiteren ist die vorliegende Erfindung nicht auf die Verwendung zweier Kulturgefäße 4 und 6 beschränkt, sondern es können auch mehrere Kulturgefäße, z.B. in Serienschaltung und/oder in Parallelschaltung angeordnet sein, so daß mehrere erste Kulturgefäße 4ᵢ und mehrere zweite Kulturgefäße 6ᵢ vorhanden sind.

In einer weiteren Ausführungsform der vorliegenden Erfindung wäre es möglich, außer dem ersten und zweiten Kulturgefäß mindestens ein weiteres Kulturgefäß vorzusehen, um z.B. ein bereits verwendetes sterilisierendes Agens 21, das jedoch nochmals verwendet werden könnte, zwischenzuspeichern. Auch für etwaige Zwischenschritte wäre es denkbar, ein weiteres Kulturgefäß vorzusehen.

## Patentansprüche

1. Vorrichtung zur Selektion beschleunigter Proliferation lebender Zellen in Suspension mit:
(a) mindestens einem ersten und mindestens einem zweiten Kulturgefäß (4, 6) zur Aufnahme einer Kultur (38);
(b) einer Gasquelle (12);
(c) einer Mediumspuelle (18);
(d) einer Quelle (20) für ein sterilisierendes Agens (21);
(e) einem Leitungssystem zum Verbinden der Gasquelle (12) mit den Kulturgefäßen (4, 6) und mit Mitteln zum wahlweisen Verbinden der beiden Kulturgefäße (4 oder 6) mit der Mediumsquelle (18) oder mit der Quelle (20) für das sterilisierende Agens (21), wobei das Leitungssystem für die Zuleitung von Gas, Medium und/oder sterilisierendem Agens in die einzelnen Kultungefaßen (4, 6) jeweils einen gemeinsamen Abschnitt (8, 10) aufweisen; und
(f) Mitteln zum Verbinden der beiden Kulturgefäße (4, 6) miteinander über zwei Verbindungsleitungen (30, 32), die einen gemeinsamen Leitungsabschnitt aufweisen.

2. Vorrichtung nach Anspruch 1, wobei die Mittel zum wahlweisen Verbinden Ventile sind.

3. Vorrichtung nach Anspruch 1 oder 2, wobei der gemeinsame Leitungsabschnitt (34) auf einem Niveau zwischen dem höchsten und tiefsten Punkt der beiden Kulturgefäße (4, 6) angeordnet ist.

4. Vorrichtung nach Anspruch 1, 2 oder 3, wobei am gemeinsamen Leitungsabschnitt (34) eine Ablaßleitung (36) vorgesehen ist, durch die Kulturen (38) aus den Kulturgefäßen (4, 6) entnehmbar sind.

5. Vorrichtung nach einem der Ansprüche 1 bis 4, wobei eine der Verbindungsleitungen (32) an einem unteren Bereich der Kulturgefäße (4, 6) und die andere der Verbindungsleitungen (30) an einem oberen Bereich der Kulturgefäße (4, 6) angeschlossen ist.

6. Vorrichtung nach einem der Ansprüche 1 bis 5, wobei die Leitungen (14, 16) von der Mediumsquelle (18) und/oder die Leitungen (22, 24) von der Quelle (20) für das sterilisierende Agens (21) in jeweils eine dem jeweiligen Kulturgefäß (4, 6) zugeordnete, von der Gasquelle (12) kommende Leitung (8, 10) münden.

7. Vorrichtung nach einem der Ansprüche 1 bis 6, wobei die Leitungen des Leitungssystems, die mit der Gasquelle (12), der Mediumsquelle (18) und/oder der Quelle (20) in Verbindung stehen, unter Überdruck stehen.

8. Vorrichtung nach einem der Ansprüche 1 bis 7, wobei eine eigene, absperrbare Ablaßleitung (26, 28) für jedes der Kulturgefäße (4, 6) vorgesehen ist.

9. Vorrichtung nach Anspruch 8, wobei die Ablaßleitungen (26, 28) aus dem oberen Bereich der Kulturgefäße (4, 6) münden und/oder von der Verbindungsleitung (30) abzweigen.

10. Vorrichtung nach einem der Ansprüche 1 bis 9, wobei mehrere erste Kulturgefäße (4ᵢ) und/oder mehrere zweite Kulturgefäße (6ᵢ) in Parallelschaltung vorgesehen sind.

11. Verfahren zur Selektion beschleunigter Proliferation lebender Zellen in Suspension mit den Schritten:
(a) Bereitstellen einer Kultur (38) in mindestens einem ersten Kulturgefäß (4);
(b) kontinuierliches Versorgen der Kultur (38) im ersten Kulturgefäß (4) mit Gas von einer Gasquelle (12) und regelmäßiges Nachliefem von Flüssigkeiten von einer Mediumsquelle (18);
(c) Überführen der Kultur (38) aus dem ersten Kulturgefäß (4) durch Verbindungsleitungen (32 - 35) in mindestens ein zweites Kulturgefäß (6) mittels entsprechender Leitungsschaltung;
(d) Verbinden des ersten Kulturgefäßes (4) mit einer Quelle (20) für ein sterilisierendes Agens (21), um das erste Kulturgefäß (4) zu sterilisieren;
(e) Entfernen des sterilisierenden Agens (21) aus dem ersten Kulturgefäß (4);
(f) kontinuierliches Versorgen der Kultur (38) im zweiten Kulturgefäß (6) mit Gas von der Gasquelle (12) und regelmäßiges Nachliefern von Flüssigkeiten von der Mediumsquelle (18);
(g) Rückführen der Kultur (38) aus dem zweiten Kulturgefäß (6) durch die Verbindungsleitungen (32 - 35) in das erste Kulturgefäß (4) mittels entsprechender Leitungsschaltung;
(h) Verbinden des zweiten Kulturgefäßes (6) mit der Quelle (20) für das sterilisierende Agens (21), um das zweite Kulturgefäß (6) zu sterilisieren; und
(i) Entfemen des sterilisierenden Agens (21) aus dem zweiten Kulturgefäß (6);

12. Verfahren nach Anspruch 11, mit wiederholen der Schritte (b) bis (h).

13. Verfahren nach Anspruch 11 oder 12, wobei die dem jeweiligen Kulturgefäß (4, 6) zugeordneten Leitungsabschnitte durch entsprechende Leitungsschaltung sterilisiert werden.

14. Verfahren nach einem der Ansprüche 11 bis 13, wobei Reste des sterilisierenden Agens (21) in den Leitungsabschnitten oder dem Kulturgefäß (4, 6) nach der Sterilisation neutralisiert werden.

15. Verfahren nach einem der Ansprüche 11 bis 14, wobei zu jedem beliebigen Zeitpunkt, zu dem kein sterilisierendes Agens (21) abgeführt wird, Kulturen (38) aus der Kulturapparatur (2) abgeführt werden.

16. Verfahren nach einem der Ansprüche 11 bis 15, wobei die Überführung der Kulturen (38) von einem Kulturgefäß (4 oder 6) in das andere Kulturgefäß (4 oder 6) periodisch erfolgt.

17. Verfahren nach einem der Ansprüche 11 bis 15, wobei das Überführen der Kulturen (38) von einem Kulturgefäß (4 oder 6) in das andere Kulturgefäß (4 oder 6) in solchen Intervallen erfolgt, daß die Population von Organismen in Suspension zu jedem Zeitpunkt erhalten bleibt und alle verdünnungsresistenten Varianten in der Apparatur während jedem dieser Zyklen zerstört werden.

18. Verfahren nach einem der Ansprüche 11 bis 17, wobei die der Kultur (38) zugeführte Flüssigkeit Nährmedien und/oder Waschlösungen aufweist.

19. Verfahren nach einem der Ansprüche 11 bis 18, wobei das Entfemen des sterilisierenden Agens (21) zumindest teilweise durch den Kulturgefäßen (4, 6) zugeordnete Ablaßleitungen (26, 28) erfolgt.

20. Verfahren nach einem der Ansprüche 11 bis 19, wobei mindestens zwei der Schritte (b) bis (h) zeitüberlappend oder zeitgleich durchgeführt werden.

21. Verfahren nach einem der Ansprüche 11 bis 20, wobei die Sterilisierung durch NaOH erfolgt.

22. Verfahren nach einem der Ansprüche 11 bis 21, wobei die Kultur unter Chemostatbedingungen oder Turbidostatbedingungen gehalten wird.

## Claims

1. A device for selecting accelerated proliferation of living cells in suspension comprising:
(a) at least a first and at feast a second culture vessel (4, 6) for receiving a culture (38);
(b) a gas source (12);
(c) a medium source (18);
(d) a source (20) for a sterilizing agent (21);
(e) a conduit system for connecting said gas source (12) with said culture vessels (4, 6) and comprising means for selectively connecting said two culture vessels (4 or 6) with said medium source (18) or with said source (20) for said sterilizing agent (21), wherein the conduit system for supplying gas, medium and/or sterilizing agent into the individual culture vessels (4, 6) each have a common portion (8, 10), and
(f) means for connecting said two culture vessels (4, 6) with each other via two connection conduits (30, 32) having a common conduit portion.

2. The device according to claim 1, wherein said means for selectively connecting are valves.

3. The device according to claim 1 or 2, wherein said common conduit portion (34) is arranged on a level between the highest and lowest points of said two culture vessels (4, 6).

4. The device according to claim 1, 2 or 3, wherein said common conduit portion (34) has an outlet conduit (36) through which said cultures (38) can be discharged from said culture vessels (4, 6).

5. The device according to any one of claims 1 to 4, wherein one of said connection conduits (32) is connected to a lower portion of said culture vessels (4, 6) and said other one of said connection conduits (30) is connected to an upper portion of said culture vessels (4, 6).

6. The device according to any one of claims 1 to 5, wherein the conduits (14, 16) from said medium source (18) and/or the conduits (22, 24) from said source (20) for said sterilizing agent (21) mount into a conduit (8, 10) which is allocated to said respective culture vessel (4, 6) and extends from said gas source (12).

7. The device according to any one of claims 1 to 6, wherein the conduits of the conduit system which are connected with said gas source (12), said medium source (18) and/or said source (20) are pressurized.

8. The device according to any one of claims 1 to 7, wherein each culture vessel (4, 6) has its own, closable outlet conduit (26, 28).

9. The device according to claim 8, wherein said outlet conduits (26, 28) mount from the upper portion of said culture vessels (4, 6) and/or branch off from said connection conduit (30).

10. The device according to any one of claims 1 to 9, wherein a plurality of first culture vessels (4ᵢ) and/or a plurality of second culture vessels (6ᵢ) being arranged in parallel are provided.

11. A method for selecting accelerated proliferation of living cells in suspension comprising the steps of:
(a) providing a culture (38) in at least one first culture vessel (4);
(b) continuously supplying said culture (38) in said first culture vessel (4) with gas from a gas source (12) and regularly delivering liquids from a medium source (18);
(c) transferring said culture (38) from said first culture vessel (4) via connection conduits (32-35) into at least one second culture vessel (6) by means of an appropriate conduit connection;
(d) connecting said first culture vessels (4) with a source (20) for a sterilizing agent (21) in order to sterilize said first culture vessel (4);
(e) removing said sterilizing agent (21) from said first culture vessel (4);
(f) continuously supplying said culture (38) in said second culture vessel (6) with gas from said gas source (12) and regularly delivering liquids from said medium source (18);
(g) transferring said culture (38) from said second culture vessel (6) via said connection conduits (32-35) back into said first culture vessel (4) by means of an appropriate conduit connection;
(h) connecting said second culture vessel (6) with said source (20) for said sterilizing agent (21) in order to sterilize said second culture vessel (6); and
(i) removing said sterilizing agent (21) from said second culture vessel (6).

12. The method according to claim 11 comprising repeating steps (b) to (h).

13. The method according to claim 11 or 12, wherein said conduit portions being allocated to said respective culture vessel (4, 6) are sterilized by an appropriate conduit connection.

14. The method according to any one of claims 11 to 13, wherein residues of said sterilizing agent (21) in said conduit portions or said culture vessel (4, 6) are neutralized after sterilization.

15. The method according to any one of claims 11 to 14, wherein at any point of time at which no sterilizing agent (21) is discharged, cultures (38) are discharged from said culture apparatus (2).

16. The method according to any one of claims 11 to 15, wherein the transfer of said cultures (38) from one culture vessel (4 or 6) into said other culture vessel (4 or 6) takes place periodically.

17. The method according to any one of claims 11 to 15, wherein the transfer of said cultures (38) from one culture vessel (4 or 6) into said other culture vessel (4 or 6) takes place in such intervals that the population of organism in suspension is maintained at any point of time and all dilution-resistant variants in said apparatus are destroyed during any one of these cycles.

18. The method according to any one of claims 11 to 17, wherein the liquid supplied to said culture (38) contains nutrient media and/or washing solutions.

19. The method according to any one of claims 11 to 18, wherein said sterilizing agent (21) is at least partially removed through outlet conduits (26, 28) allocated to said culture vessels (4, 6).

20. The method according to any one of claims 11 to 19, wherein at least two of said steps (b) to (h) overlap temporarily or take place at the same time.

21. The method according to any one of claims 11 to 20, wherein NaOH is used for the sterilization.

22. The method according to any one of claims 11 to 21, wherein the culture is held under chemostat conditions or turbidostat conditions.

## Revendications

1. Procédé pour la sélection de la prolifération accélérée de cellules vivantes en suspension, comprenant:
a) au moins une première et au moins une deuxième cuve de culture (4, 6) destinées à recevoir une culture (38),
b) une source de gaz (12),
c) une source de milieu (18),
d) une source (20) d'un agent de stérilisation (21),
e) un système de tuyaux destiné à relier la source de gaz (12) aux cuves de culture (4, 6) et disposant de moyens pour relier, au choix, les deux cuves de culture (4 ou 6) à la source de milieu (18) ou à la source (20) de l'agent de stérilisation (21), où le système de tuyaux présente à chaque fois une section commune (8, 10) pour l'arrivée de gaz, de milieu et / ou d'agent stérilisant dans les cuves de culture individuelles (4, 6) et
f) des moyens pour relier les deux cuves de culture (4, 6) entre elles via deux tuyaux de liaison (30, 32) qui présentent une section de tuyauterie commune.

2. Dispositif selon la revendication 1, où les moyens de liaison au choix sont des vannes.

3. Dispositif selon la revendication 1 ou 2, où la section de tuyauterie commune (34) est installée à un niveau situé entre le point le plus haut et le point le plus bas des deux cuves de culture (4, 6).

4. Dispositif selon la revendication 1, 2 ou 3, où il est prévu sur la section de tuyauterie commune (34) une ligne de soutirage (36) permettant le prélèvement de la culture (38) sur les cuves de cultur (4,6).

5. Dispositif selon l'une des revendications 1 à 4, où l'un des tuyaux de liaison (32) est raccordé à une zone inférieure des cuves de culture (4, 6) et l'autre tuyau de liaison (30) à une zone supérieure des cuves de culture (4,6).

6. Dispositif selon l'une des revendications 1 à 5, où les tuyaux (14, 16) pour la source de milieu (18) et / ou les tuyaux (22, 24) de la source (20) pour l'agent de stérilisation (21) débouchent dans une ligne entrante (8, 10) provenant de la source de gaz (12) et reliée à chaque fois à l'une des cuves de culture (4,6).

7. Dispositif selon l'une des revendications 1 à 6, où les lignes du système de tuyaux, reliées à la source de gaz (12), à la source de milieu (18) et / ou à la source (20), sont sous pression.

8. Dispositif selon l'une des revendications 1 à 7, où il est prévu pour chacune des cuves de culture (4,6) une ligne de décharge (26, 28) qui peut être fermée.

9. Dispositif selon la revendication 8, où les lignes de décharge (26, 28) sortent de la partie supérieure des cuves de culture (4, 6) et / ou dérivent du tuyau de liaison (30).

10. Dispositif selon l'une des revendications 1 à 9, où il est prévu en parallèle plusieurs premières cuves de culture (4ᵢ) et / ou plusieurs deuxièmes cuves de culture (6ᵢ).

11. Procédé de sélection de la prolifération accélérée de cellules vivantes en suspension comportant les étapes suivantes:
(a) chargement d'une culture (38) dans au moins une première cuve de culture (4),
(b) alimentation en continu de la culture (38) dans la première cuve de culture (4) par un gaz provenant d'une source de gaz (12) et appoint régulier de liquides provenant d'une source de milieu (18),
(c) transfert de la culture (38) de la première cuve de culture (4), par des tuyaux de liaison (32 - 35) dans au moins une deuxième cuve de culture (6) par une commutation appropriée du circuit de tuyaux,
(d) raccordement de la première cuve de culture (4) à une source (20) d'agent de stérilisation (21), afin de stériliser la première cuve de culture (4),
(e) élimination de l'agent de stérilisation (21) de la première cuve de culture (4),
(f) alimentation en continu de la culture (38) dans la deuxième cuve de culture (6) avec du gaz provenant de la source de gaz (12) et appoint régulier de liquides à partir de la source de milieu (18),
(g) renvoi de la culture (38) de la deuxième cuve de culture (6), par les tuyaux de liaison (32 - 35), dans la première cuve de culture (4) par une commutation appropriée du circuit de tuyaux,
(h) raccordement de la deuxième cuve de culture (6) à la source (20) pour l'agent de stérilisation (21), afin de stériliser la deuxième cuve de culture (6), et
(i) élimination de l'agent de stérilisation (21) de la deuxième cuve de culture (6).

12. Procédé selon la revendication 11, avec répétition des étapes (b) à (h).

13. Procédé selon la revendication 11 ou 12, où les sections de tuyaux respectivement coordonnées aux cuves de culture (4,6) sont stérilisées par une commutation appropriée du circuit de tuyaux.

14. Procédé selon l'une des revendications 11 à 13, où les résidus de l'agent de stérilisation (21) sont neutralisés dans les sections de tuyaux ou les cuves de culture (4,6) après la stérilisation.

15. Procédé selon l'une des revendications 11 à 14 où, à tout moment auquel de l'agent de stérilisation (21) n'est pas soutiré, de la culture (38) est soutirée de l'appareil de culture (2).

16. Procédé selon l'une des revendications 11 à 15, où le transfert de la culture (38) d'une cuve de culture (4 ou 6) dans l'autre cuve de culture (4 ou 6) est opéré de manière périodique.

17. Procédé selon l'une des revendications 11 à 15, où le transfert de la culture (38) d'une cuve de culture (4 ou 6) dans l'autre cuve de culture (4 ou 6) est opéré à des intervalles tels que la population d'organismes en suspension demeure maintenue à tout moment et que toutes les variantes résistantes à la dilution soient détruites dans l'appareil pendant ces cycles.

18. Procédé selon l'une des revendications 11 à 17, où le liquide envoyé à la culture (38) contient des nutriments et / ou des solutions de lavage.

19. Procédé selon l'une des revendications 11 à 18, où l'élimination de l'agent de stérilisation (21) est opérée au moins en partie via les lignes de soutirage (26, 28) coordonnées aux cuves de culture (4,6).

20. Procédé selon l'une des revendications 11 à 19, où au moins deux des étapes (b) à (h) sont entreprises avec chevauchement dans le temps ou simultanément.

21. Procédé selon l'une des revendications 11 à 20, où la stérilisation est opérée par du NaOH.

22. Procédé selon l'une des revendications 11 à 21, où la culture est maintenue dans des conditions de chémostat ou de turbidostat.
